Europäisches Patentamt

European Patent Office

Office européen des brevets

(11) Publication number: **0 347 748 B1**

## EUROPEAN PATENT SPECIFICATION

(45) Date of publication of patent specification: **19.11.92** (51) Int. Cl.5: **A61K 9/52**, A61K 9/20

(21) Application number: **89110864.9**

(22) Date of filing: **15.06.89**

(54) **Pharmaceutical granules and tablets made therefrom.**

(30) Priority: **24.06.88 US 211495**
**22.05.89 US 353809**

(43) Date of publication of application:
**27.12.89 Bulletin 89/52**

(45) Publication of the grant of the patent:
**19.11.92 Bulletin 92/47**

(84) Designated Contracting States:
**AT BE CH DE ES FR GB GR IT LI LU NL SE**

(56) References cited:
**EP-A- 0 196 546**
**WO-A-86/04817**
**FR-A- 2 392 667**
**US-A- 4 533 674**
**US-A- 4 555 399**

(73) Proprietor: **ABBOTT LABORATORIES
CHAD-0377, AP6D/2, One Abbott Park Road
Abbott Park, Illinois 60064-3500(US)**

(72) Inventor: **Brinker, Dale R.
453 Linden Lane
Antioch Illinois 60002(US)**
Inventor: **Reiland, Thomas L.
33974 North Lake Shore Drive
Gages Lake Illinois 60030(US)**

(74) Representative: **Modiano, Guido et al
c/o Modiano & Associati S.r.l. Via Meravigli,
16
I-20123 Milano(IT)**

## Description

Technical Field

This is a continuation-in-part of U.S. patent application Serial No. 211,495, filed June 24, 1988. This invention relates to pharmaceutical granulations and tablet dosage forms. In particular, it relates to coated pharmaceutical granules and tablets made from such granules.

Background Art

In the manufacture of pharmaceutical products, the active pharmaceutical agent is combined with the desired excipients, diluents and other adjuvant materials in a liquid environment and granulated by conventional techniques to produce active drug granules of a size suitable for incorporation in the desired finished product. For tablet products, for example, a granule size of from 0.6 to 2.5 mm (10 to 40 mesh) may be preferred. In tabletting, the granules are metered into a tablet press and compressed under high pressures to form a cohesive tablet having a certain required degree of physical integrity, surface smoothness, and other physical properties known to the art. The resulting tablet can then be film- or enteric-coated if desired to provide the desired color, flavor, mouthfeel, bioavailability, resistance to abrasion, etc.

Controlled delivery of drugs from pharmaceutical tablets frequently involves the use of coatings to impart acid- or enzyme-resistance, delayed release, and other desirable release properties. A preferred method of employing such coatings is to directly coat a granulation of the desired pharmaceutical active ingredient. Such granules can be almost entirely active drug, or can be built up from nonpareil seeds, or by other techniques readily familiar to those of skill in the pharmaceutical manufacturing arts.

A difficulty is encountered in compressing such coated granules into commercially usable tablet products. Such granules can be formed into relatively soft tablets using low compression forces. However, the compressive forces required to produce a tablet which is sufficiently strong and cohesive to survive the stresses imposed by the subsequent film-coating process and commercial packaging and distribution inevitably result in fracture of the friable coating on a substantial percentage of the granules, resulting in uncontrolled rather than controlled release of the drug.

It has been known in the prior art to incorporate such coated granules into a tablet matrix which further incorporates a material which stabilizes the granules against compressive loads, such as disclosed in European Patent Application 194,546 of Becker. In that application, microcrystalline cellulose is used to form a matrix which effectively distributes compressive loads through the tablet as it is being formed, in effect bypassing the granules as load bearing elements of the tablet during compression.

A problem remains, however, in compressing granules formed from pharmaceutical agents whose physical properties cause the granule to be relatively smooth in surface texture or relatively waxy in overall granule texture. With such agents, such as divalproex sodium, the smooth granule surfaces make it difficult or impossible to achieve acceptable cohesion in tablets formed from the granules, even when very high compression forces are employed. Enteric and other coatings tend to make the surfaces of such smooth granules even smoother, as well as harder, further complicating this problem.

It is an object of this invention to provide a method of tableting drug granules having smooth surfaces which provides acceptable cohesiveness in the resulting tablets.

It is another object of this invention to provide granules which, although made from a material which is difficult to compress into tablets, can be made into tablets having acceptable cohesiveness.

It is a further object of this invention to provide granules and methods which accomplish the foregoing objects while avoiding fracture of any enteric or other coatings on the tablets. These and other objects of the invention will be evident from the following disclosure.

WO-A-8 604 817 discloses a pharmaceutical dosage unit comprising potassium chloride crystals coated with a controlled release coat of ethylcellulose and hydroxypropylcellulose or polyethylene glycol and further comprising microcrystalline cellulose admixed in the tablet formulation. No compression enhancing action is attributed to the content of microcrystalline cellulose in the tablet matrix.

Derwent abstract 72723A/41 (GB1598458) discloses a pharmaceutical tablet formulation containing enteric coated granules and microcrystalline cellulose. However, the tablet formulation also contains a polymer or waxy substance to which a granule-protecting activity is ascribed. The abstract in no way attributes the granule-protective action to the content of microcrystalline cellulose. Further, the microcrystalline cellulose is simply admixed in the tablet formulation.

## Disclosure of the Invention

This invention provides pharmaceutical granules and tablets made therefrom. In particular, it provides a granule comprising an active drug, wherein the granule has a compression-enhancing coating comprising a polymer selected from povidone, hydroxypropyl cellulose and hydroxypropyl methylcellulose, and microcrystalline cellulose. Preferrably, the coating as applied consists essentially of an ethanol (or any other suitable solvent system) solution of from 0.5% to 10% (w/v) povidone and from 5% to 25% (w/v) microcrystalline cellulose. In the final, spray-coated granules, i.e., after the ethanol has evaporated, the coating contains from about a 1:15 to about 2:1 by weight povidone: microcrystalline cellulose. Especially preferred as the coating solution is a U.S.P. ethanol solution of 1% povidone K-90 (w/v) and 10% microcrystalline cellulose (w/v). "w/v" as used herein means weight per unit volume of liquid (i.e., grams/liter). While not intending to be limited by theory, it is thought that the polymer material functions as a binder and carrier for the microcrystalline cellulose, while the microcrystalline cellulose itself imparts the excellent compressibility properties to the granules that microcrystalline cellulose is well known for.

The active ingredient in the granules can be any drug accepted for use in pharmaceutical tablet products. Such drugs are well known to those of ordinary skill in the pharmaceutical manufacturing arts. The granules can be made using a single drug or a mixture of drugs, or a mixture of different granules, each containing one or more drugs, can be used. An additional enteric coating, acid-resistant coating, microporous coating, or other coating intended to control the release rate or dissolution rate of the drug granule can be applied to the granule before the compression enhancing coating is applied. Among the materials useful for this purpose are acrylic polymers and copolymers, ethylcellulose, hydroxypropylcellulose, hydroxypropylmethylcellulose, polyvinyl acetate, polyvinyl acetate phthalate, cellulose acetate phthalate, hydroxypropylmethylcellulose phthalate, zein, shellac, acacia, nylon, sugar, anionic acrylic resins, and the like.

The microcrystalline cellulose used in the practice of this invention is an article of commerce, available from a variety of sources, and is a National Formulary material. Its manufacture is described by Battista, Ind. Eng. Chem., 42, 502 (1950) and U.S. Patents 2,978,446 and 3,141,875. It is a nonfibrous powder having the particulate form of rigid rods and a bulk density of 288-304 kg/m$^3$ (18 to 19 pounds per cubic foot). It is practically insoluble in water, but is dispersible therein.

Additional tableting aids, excipients, binders, disintegrants, lubricants, fillers, etc., well known to the pharmaceutical arts can also be employed at minor levels (generally less than 10%, preferably less than 2%) in the practice of this invention. Such inert additives include a variety of stearates, tableting aids, starches, gums, waxes, silicates, polymers and the like. Microcrystalline cellulose can also be employed as a tableting aid apart from its use in the compression-enhancing granule coating of this invention.

If desired, uncoated granules of drug can also be included in the table matrix. Just as the coated granules in a given tablet can be made from a single drug or a number of drugs, the uncoated granules optionally incorporated in the tablet matrix can be the same drug or drugs used in the coated granules, or they may be a different drug or mixture of drugs, as dictated by the desires of the formulator.

The compression enhancing particle coating of this invention can be applied by numerous conventional granule coating techniques. For example, the coating can be applied by spraying in a Wurster-type fluidized bed coating apparatus, or by conventional microencapsulation techniques. In a small scale particle coating process, the following operating conditions have been found to be useful: an atomizing air pressure of about 4 atmospheres, an inlet air temperature of about 50°C, a spray rate of about 70 grams/minute for a 3 kg batch of granules, to provide an outlet temperature of from 28° to 35°C. At lower temperatures or higher feed rates, overwetting of the particles can occur, while at higher temperatures spray drying of the coating into discrete particles can take place. The resulting coated particles have a core of pharmaceutical active ingredient, and a surface coating of povidone and microcrystalline cellulose. These particles can then be used in any conventional tablet formulation with improved tableting results.

The following Examples illustrate the practice of this invention, without intending to be limitative thereof.

## Example 1

Nonpareils were coated with an enteric coating liquid made by dissolving 10% hydroxypropyl methylcellulose phthalate (HP-55), 1% triethyl citrate, and 0.02% dye, blue, FD&C No. 2 in a mixture of 80/20 acetone and 200 proof alcohol, SD 3A. The nonpareils were coated with this coating liquid in a Glatt GPCG-5 Wurster Column Coater. After the coating was applied, 4.5L of a compression-enhancing coating was spray applied to coated batch in the GPCG-5 Particle Coater. The formula for the compression-enhancing coating comprises 1% (w/v) povidone, USP (K-Value 90) and 10% (w/v) microcrystalline cellulose, (Avicel PH 105) in 200 proof alcohol, SD 3A.

The coated (double) nonpareils were then compressed in the following blend:

| Item | mg/tablet |
|---|---|
| 1. Lactose, Monohydrate, NF, Powder Regular | 40.0 |
| 2. Cellulose, Microcrystalline, NF (Avicel 101) | 290.0 |
| 3. Crospovidone, NF | 15.0 |
| 4. Hydroxypropyl Cellulose, NF | 10.0 |
| 5. Coated Nonpareils | 840.00 |
| 6. Acid, Stearic, NF, Fine Powder | 4.0 |
| 7. Colloidal Silicon Dioxide, NF (Cab-O-Sil) | 1.0 |

The blend was compressed to a tablet weight of 1200 mg.

Example 2

249.6 kg of divalproex sodium was blended with 10.4 kg of silica gel in a twin shell blender and milled using a Fitzmill, medium speed knives forward, though a 2A band. This blend was then granulated with 16 liters of alcohol (SD 3A, 200 proof) in a Gral High Intensity Granulator and then dried in an Areomatic Fluid Bed Dryer at an exhaust temperature of 50°C for 30 minutes. The dried granulation was then sifted through 12 and 24 mesh screens using a Sweco Sifter and the larger than 2.1 mm diameter (12 mesh) material was milled using a Fitzmill, as above and then resifted.

The 206 kg of 2.1-4.2 mm (12-24 mesh) particles were then coated in a Glatt CPCG-45 Wurster Coating Column using the following coating liquids:

1) 330 liters of a coating liquid prepared by dispersing 19.8 kg of ethylcellulose (NF, 7cps), 3.3 kg of triethyl citrate (Citroflex-2), and 19.8 kg of magnesium stearate (NF, impalpable powder) in a mixture of 66 liters of alcohol (SD 3A, 200 proof) and acetone (used to bring the volume of liquid to 330 liters).

2) 413 liters of a coating liquid prepared by dispersing 41.3 kg of hydroxypropyl methylcellulose phthalate (HP-55), 4.13 kg of triethyl citrate and 826 g of dye (blue, FD&C No. 2) in a mixture of 82.6 liters of alcohol (SD 3A, 200 proof) and acetone (used to bring the volume of livid to 413 liters).

3) 310 liter of the Compression Enhancing Coating Liquid prepared by dispersing 3.1 kg of povidone (K-Value 90) and 31 kg of microcrystalline cellulose (Avicel PH 105) in alcohol (SD 3A, 200 proof).

The coating conditions used for each of the coating liquids were as follows:

| Coating Liquid | 1 | 2 | 3 |
|---|---|---|---|
| Inlet Air Temperature (°C) | 52 | 50 | 52 |
| Relative Humidity (%) | 15 | 15 | 15 |
| Atomization Air Pressure (Pa)/(PSIG) | $5.5.10^5$/65 | $5.5.10^5$/65 | $3.4.10^5$/35 |
| Solution Flow Rate (ml/min/nozzle) | 320 | 280 | 500 |
| (NOTE: This unit contains 7 nozzles) | | | |

The coated particles are then discharged and sifted through a 2.5 mm (10 mesh) screen in a Sweco Sifter.

The smaller than 2.5 mm (10 mesh) particles were then blended with the following items in a twin shell blender and compressed on a Fette 2000 compressing machine.

| Item | mg/tablet |
|---|---|
| 1. Divalproex Sodium Coated Particles | 893.2 |
| 2. Lactose, Monohydrate, NF Powder Regular | 50.0 |
| 3. Cellulose, Microcrystalline, NF (Avicel PH101) | 291.2 |
| 4. Crospovidone, NF | 15.0 |
| 5. Hydroxypropyl Cellulose, NF | 10.0 |
| 6. Acid, Stearic, NF, Fine Powder | 5.0 |
| 7. Colloidal Silicon Dioxide, NF (Cab-O-Sil M-5) | 1.2 |

These tablets were then coated in an Accela-Cota (at an exhaust temperature of 35°C, atomization air pressure of $5.2.10^5$ Pa (60 psig), liquid spray rate of approximately 700g/min and pan rotating speed of 6 rpm) using 300 ml/kg of tablets with the following coating liquid:

| Item | % w/w |
|---|---|
| 1. Water, Purified, USP (Distilled) | 10.0% |
| 2. Alcohol, SD 3A, 200 Proof | q.s. |
| 3. Hydroxypropyl Methylcellulose 2910, USP, 6CPS | 4.0% |
| 4. Propylene Glycol, USP | 0.8% |
| 5. Vanillin, NF, Crystals | 0.3% |

## Example 3

4 kg of erythromycin base was blended with 160 g of microcrystalline cellulose (Avicel PH 101), hydroxypropyl methylcellulose (2910 USP, 15 CPS), and povidone (K-Value 90) in a twin shell blender. This blend was then granulated with 900 ml of distilled water and 50 g of polyethylene glycol 400 in a planetary mixer and extruded through a 0.8 mm band at a speed of 0.4. The extruded material was then spheronized at a speed of 0.7 and then oven dried at 50°C overnight. The dried granulation was then sifted through 0.85 and 1.6 mm mesh size (16 and 30 mesh) screens using a Sweco Sifter.

The 4 kg of 0.85-1.6 mm (16-30 mesh) particles were then coated in a Glatt CPCG-5 Wurster Coating Column using the following coating liquids:

1. 12 liters of a coating liquid prepared by dispersing 120 g of acetyl tributyl citrate (Citroflex A-4), 1 kg of hydroxypropyl methylcellulose phthalate (HP-50) and 18 g of dye red D&C No. 30 lake in a mixture of 4.8 liters of alcohol (SD 3A, 200 proof) and acetone (used to bring the volume of liquid to 12 liters).

2. 4 liters of the Compression Enhancing Coating Liquid prepared by dispersing 80 g of hydroxypropyl methylcellulose (2910, USP, 15 CPS), 80 g of hydroxypropyl cellulose, 16 g of propylene glycol, and 400 g of microcrystalline cellulose (Avicel PH 101) in alcohol (SD 3A, 200 proof).

The particles were then blended with the following items in a twin shell blender and compressed on a Fette 1000 compressing machine.

| Item | mg/tablet |
|---|---|
| 1. Erythromycin Coated Particles | 984.3 |
| 2. Cellulose, Microcrystalline, NF (Avicel PH 101) | 102.9 |
| 3. Crospovidone, NF | 102.9 |
| 4. Magnesium Stearate, NF, Impalpable Powder | 2.5 |
| 5. Colloidal Silicon Dioxide, NF (Cab-O-Sil M-5) | 2.5 |
| 6. Talc, USP Powder | 2.5 |
| 7. Wax, Hydrogenated Vegetable Oil (Sterotex K) | 2.5 |

These tablets were then coated in an Accela-Cota (at an exhaust temperature of 60°C, atomization air pressure of $5.2.10^5$ Pa (60 psig), and pan rotating speed of 8 rpm) using 300 ml/kg of tablets with a clear gloss solution.

Compression Profile of Coated Particles

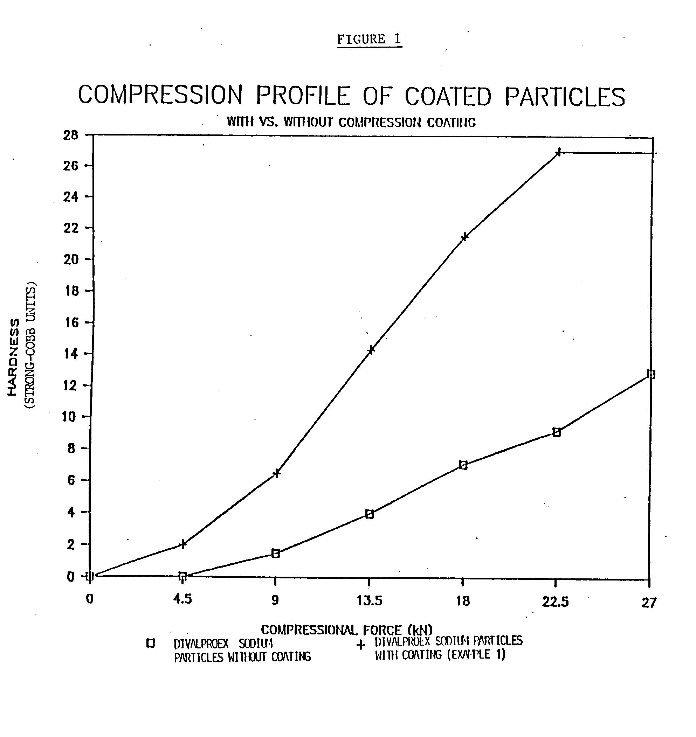

The attached graph (Figure 1) shows a compressional force versus hardness (measured in Strong-Cobb units) profile for divalproex sodium particles which have been coated with the compression-enhancing coating of Example 1 and divalproex sodium particles which have not been coated with the compression-enhancing coating. The data presented in Figure 1 was obtained using a Strong-Cobb type hardness tester. For the particles with the compression-enhancing coating the hardness data points for 22.5 and 27 kiloNewtons (kN) of compressional force was beyond the capacity of the hardness measuring equipment (>27), but were plotted as hardness of 27.

The graph demonstrates that particles coated with compression-enhancing coating can (1) withstand a greater compressional force and, therefore, are less likely to rupture or fracture, and (2) be made into tablets using a lower compressional force than when a compression-enhancing coating is not used.

Tablets in accordance with this invention having a total weight of about 100 mg, and the coated drug granules contained therein, should have a hardness of at least about 4-5 Strong-Cobb units. Tablets in accordance with this invention having a total weight of about 500-600 mg, and the coated drug granules contained therein, should have a hardness of at least about 10 Strong-Cobb units. Tablets in accordance with this invention having a total weight of about 1000 mg, and the coated drug granules contained therein, should have a hardness of at least about 14 Strong-Cobb units.

The foregoing specification including the examples and formulations are merely illustrative of the invention.

## Claims

**Claims for the following Contracting States : AT, BE, CH, DE, FR, GB, IT, LI, LU, NL, SE**

1. A composition for coating drug granules which comprises a solution of from 0.5% to 10% (w/v) of a polymer selected from povidone, hydroxypropyl cellulose and hydroxypropyl methylcellulose, and from 5% to 25% (w/v) microcrystalline cellulose.

2. The composition of Claim 1 comprising 1% povidone and 10% microcrystalline cellulose.

3. A granule for compression into tablets having a hardness sufficient to produce a cohesive tablet that will not fracture during tablet coating, comprising a core of active drug and a surface coating comprising a polymer selected from povidone, hydroxypropyl cellulose and hydroxypropyl methylcellulose and microcrystalline cellulose, the polymer:microcrystalline cellulose ratio being from 1:15 to 2:1 by weight.

4. The granule of Claim 3 wherein the active drug is divalproex sodium.

5. A tablet having a hardness sufficient to produce a cohesive tablet that will not fracture during tablet coating, and comprising granules according to claim 3.

6. The tablet of claim 5 wherein the active drug is divalproex sodium.

7. The tablet of claim 5 wherein the surface coating comprises 1% (w/v) povidone and 10% (w/v) microcrystalline cellulose.

8. The granule of Claim 3 wherein the active drug is erythromycin.

9. The tablet of Claim 5 wherein the active drug is erythromycin.

10. A granule for compression into tablets having a hardness sufficient to produce a cohesive tablet that will not fracture during tablet coating, comprising a core of divalproex sodium and a surface coating comprising povidone and microcrystalline cellulose in a ratio of about 1:10 by weight.

11. A tablet having a hardness sufficient to produce a cohesive tablet that will not fracture during tablet coating and comprising drug granules according to Claim 10.

12. A granule for compression into tablets having a hardness sufficient to produce a cohesive tablet that will not fracture during tablet coating comprising a core of erithromycin and a surface coating comprising hydroxypropyl methylcellulose, hydroxypropyl cellulose and microcrystalline cellulose in a ratio of about 1:1:5 by weight.

13. A tablet having a hardness sufficient to produce a cohesive tablet that will not fracture during tablet coating and comprising drug granules according to Claim 12.

**Claims for the following Contracting States : ES, GR**

1. A composition for coating drug granules which comprises a solution of from 0.5% to 10% (w/v) of a polymer selected from povidone, hydroxypropyl cellulose and hydroxypropyl methylcellulose, and from 5% to 25% (w/v) microcrystalline cellulose.

2. The composition of Claim 1 comprising 1% povidone and 10 % microcrystalline cellulose.

3. Use of the composition of Claims 1 for coating drug granules.

4. The use of Claim 3 wherein said drug granule is selected from divalproex sodium and erythromycin.

5. A method for making coated drug granules for compression into tablets comprising coating a drug granule with a mixture of a polymer selected from povidone, hydroxypropyl cellulose and hydroxypropyl methylcellulose and microcrystalline cellulose wherein the polymer to microcrystalline cellulose ratio is from 1:15 to 2:1.

6. The method of Claim 5 wherein said drug granule is selected from divalproex sodium and erythromycin.

7. The method of Claim 5 comprising coating a drug granule having a core of divalproex sodium with a mixture comprising povidone and microcrystalline cellulose in a ratio of about 1:10 by weight.

8. The method of Claim 5 comprising coating a drug granule having a core of erythromycin with a mixture comprising hydroxypropyl methylcellulose, hydroxypropyl cellulose and microcrystalline cellulose in a ratio of about 1:1:5.

**Patentansprüche**
**Patentansprüche für folgende Vertragsstaaten : AT, BE, CH, DE, FR, GB, IT, LI, LU, NL, SE**

1. Zusammensetzung zum Beschichten von Arzneimittelgranula, welches umfaßt eine Lösung von 0,5 % bis 10 % (Gew./Vol.) eines Polymers ausgewählt aus Povidon, Hydroxypropylcellulose und Hydroxypropylmethylcellulose und von 5 % bis 25 % (Gew./Vol.) mikrokristalliner Cellulose.

2. Zusammensetzung nach Anspruch 1, die 1 % Povidon und 10 % mikrokristalline Cellulose umfaßt.

3. Granulum zum Pressen in Tabletten mit einer Härte ausreichend, um eine kohäsive Tablette herzustellen, die während des Beschichtens der Tablette nicht bricht, umfassend einen Kern aus aktivem Arzneimittel und eine Oberflächenbeschichtung umfassend ein Polymer ausgewählt aus Povidon, Hydroxypropylcellulose und Hydroxypropylmethylcellulose und mikrokristalliner Cellulose, wobei das Gewichtsverhältnis Polymer:mikrokristalliner Cellulose von 1:15 bis 2:1 beträgt.

4. Granulum nach Anspruch 3, worin das aktive Arzneimittel Divalproex-Natrium ist.

5. Tablette mit einer Härte ausreichend, um eine kohäsive Tablette herzustellen, die während des Beschichtens der Tablette nicht bricht, und die Granula nach Anspruch 3 umfaßt.

6. Tablette nach Anspruch 5, worin das aktive Arzneimittel Divalproex-Natrium ist.

7. Tablette nach Anspruch 5, worin die Oberflächenbeschichtung 1 % (Gew./Vol.) Povidon und 10 % (Gew./Vol.) mikrokristalline Cellulose umfaßt.

8. Granulum nach Anspruch 3, worin das aktive Arzneimittel Erythromycin ist.

**9.** Tablette nach Anspruch 5, worin das aktive Arzneimittel Erythromycin ist.

**10.** Granulum zum Pressen in Tabletten mit einer Härte ausreichend, um eine kohäsive Tablette herzustellen, die während des Beschichtens der Tablette nicht bricht, umfassend einen Kern aus Divalproex-Natrium, und eine Oberflächenbeschichtung, umfassend Povidon und mikrokristalline Cellulose in einem Gewichtsverhältnis von etwa 1:10.

**11.** Tablette mit einer Härte ausreichend, um eine kohäsive Tablette herzustellen, die während des Beschichtens der Tablette nicht bricht, und die Arzneimittelgranula nach Anspruch 10 umfaßt.

**12.** Granulum zum Pressen in Tabletten mit einer Härte ausreichend, um eine kohäsive Tablette herzustellen, die während des Beschichtens der Tablette nicht bricht, umfassend einen Kern aus Erythromycin und eine Oberflächenbeschichtung umfassend Hydroxypropylmethylcellulose, Hydroxypropylcellulose und mikrokristalline Cellulose in einem Gewichtsverhältnis von etwa 1:1:5.

**13.** Tablette mit einer Härte ausreichend, um eine kohäsive Tablette herzustellen, die während des Beschichtens der Tablette nicht bricht, und die Arzneimittelgranula nach Anspruch 12 umfaßt.

**Patentansprüche für folgende Vertragsstaaten : ES, GR**

**1.** Zusammensetzung zum Beschichten von Arzneimittelgranula, welches umfaßt eine Lösung von 0,5 % bis 10 % (Gew./Vol.) eines Polymers ausgewählt aus Povidon, Hydroxypropylcellulose und Hydroxypropylmethylcellulose und von 5 % bis 25 % (Gew./Vol.) mikrokristalline Cellulose.

**2.** Zusammensetzung nach Anspruch 1, umfassend 1 % Povidon und 10 % mikrokristalline Cellulose.

**3.** Verwendung der Zusammensetzung nach Anspruch 1 zum Beschichten von Arzneimittelgranula.

**4.** Verwendung nach Anspruch 3, worin das Arzneimittelgranulum ausgewählt ist aus Divalproex-Natrium und Erythromycin.

**5.** Verfahren zun Herstellen von Arzneimittelgranula zum Pressen in Tabletten, umfassend Beschichten eines Arzneimittelgranulums mit einem Gemisch aus einem Polymer ausgewählt aus Povidon, Hydroxypropylcellulose und Hydroxypropylmethylcelluose und mikrokristalliner Cellulose, worin das Verhältnis von Polymer zu mikrokristalliner Cellulose von 1:15 bis 2:1 beträgt.

**6.** Verfahren nach Anspruch 5, worin das Arzneimittelgranulum ausgewählt ist aus Divalproex-Natrium und Erythromycin.

**7.** Verfahren nach Anspruch 5, umfassend Beschichten eines Arzneimittelgranulums, das einen Kern von Divalproex-Natrium hat, mit einem Gemisch umfassend Povidon und mikrokristalline Cellulose in einem Gewichtsverhältnis von etwa 1:10.

**8.** Verfahren nach Anspruch 5, umfassend Beschichten eines Arzneimittelgranulums, das einen Kern von Erythromycin hat, mit einem Gemisch umfassend Hydroxypropylmethylcellulose, Hydroxypropylcellulose und mikrocristalline Cellulose in einem Gewichtsverhältnis von etwa 1:1:5.

**Revendications**
**Revendications pour les Etats contractants suivants : AT, BE, CH, DE, FR, GB, IT, LI, LU, NL, SE**

**1.** Composition d'enrobage de granules de médicament comprenant une solution de 0,5 % à 10 % (p/v) d'un polymère sélectionné parmi la polyvidone, l'hydroxypropylcellulose et l'hydroxypropylméthylcellulose et de 5 % à 25 % (p/v) de cellulose microcristalline.

**2.** Composition selon la revendication 1, comprenant 1 % de polyvidone et 10 % de cellulose microcristalline.

**3.** Granule destiné à la compression en comprimés, ayant une dureté suffisante pour produire un comprimé cohésif qui ne se brisera pas durant l'enrobage du comprimé, comprenant un noyau de médicament actif et un enrobage superficiel comprenant un polymère sélectionné parmi la polyvidone, l'hydroxypropylcellule et l'hydroxypropylméthylcellulose et de la cellulose microcristalline, le rapport polymère : cellulose microcristaline étant compris entre 1:15 et 2:1 en poids.

4. Granule selon la revendication 3, dans lequel le médicament actif est le divalproex sodium.

5. Comprimé ayant une dureté suffisante pour produire un comprimé cohésif qui ne se brisera pas durant l'enrobage du comprimé et comprenant des granules selon la revendication 3.

6. Comprimé selon la revendication 5, dans lequel le médicament actif est le divalproex sodium.

7. Comprimé selon la revendication 5, dans lequel l'enrobage superficiel comprend 1 % (p/v) de polyvidone et 10 % (p/v) de cellulose microcristalline.

8. Granule selon la revendication 3, dans lequel le médicament actif est l'érythromycine.

9. Comprimé selon la revendication 5, dans lequel le médicament actif est l'érythromycine.

10. Granule destiné à la compression en comprimés ayant une dureté suffisante pour produire un comprimé cohésif qui ne se brisera pas durant l'enrobage du comprimé, comprenant un noyau de divalproex sodium et un enrobage superficiel comprenant de la polyvidone et de la cellulose microcristalline en un rapport en poids d'environ 1:10.

11. Comprimé ayant une dureté suffisante pour produire un comprimé cohésif qui ne se brisera pas durant l'enrobage du comprimé et comprenant des granules de médicament selon la revendication 10.

12. Granule destiné à la compression en comprimés ayant une dureté suffisante pour produire un comprimé cohésif qui ne se brisera pas durant l'enrobage du comprimé comprenant un noyau d'érythromycine et un enrobage superficiel comprenant de l'hydroxypropylméthylcellulose, de l'hydroxypropylcellulose et de la cellulose microcristalline en un rapport en poids d'environ 1:1:5.

13. Comprimé ayant une dureté suffisante pour produire un comprimé cohésif qui ne se brisera pas durant l'enrobage du comprimé et comprenant des granules de médicament selon la revendication 12.

**Revendications pour les Etats contractants suivants : ES, GR**

1. Composition d'enrobage de granules de médicament comprenant une solution de 0,5 % à 10 % (p/v) d'un polymère sélectionné parmi la polyvidone, l'hydroxypropylcellulose et l'hydroxypropylméthylcellulose et de 5 % à 25 % (p/v) de cellulose microcristalline.

2. Composition selon la revendication 1, comprenant 1 % de polyvidone et 10 % de cellulose microcristalline.

3. Emploi de la composition selon la revendication 1 pour l'enrobage de granules de médicament.

4. Emploi selon la revendication 3, dans lequel ledit granule de médicament est sélectionné parmi le divalproex sodium et l'érythromycine.

5. Procédé de préparation de granules de médicament enrobés destinés à la compression en comprimés comprenant l'enrobage d'un granule de médicament à l'aide d'un mélange d'un polymère sélectionné parmi la polyvidone, l'hydroxypropylcellulose et l'hydroxypropylméthylcellulose et de cellulose microcristalline, le rapport du polymère à la cellulose microcristalline étant compris entre 1:15 et 2:1.

6. Procédé selon la revendication 5, dans lequel ledit granule de médicament est sélectionné parmi le divalproex sodium et l'érythromycine.

7. Procédé selon la revendication 5, comprenant l'enrobage d'un granule de médicament ayant un noyau de divalproex sodium par un mélange comprenant de la polyvidone et de la cellulose microcristalline en un rapport en poids d'environ 1:10.

8. Procédé selon la revendication 5, comprenant l'enrobage d'un granule de médicament ayant un noyau d'érythromycine par un mélange comprenant de l'hydroxypropylméthylcellulose, de l'hydroxypropylcellulose et de la cellulose microcristalline en un rapport d'environ 1:1:5.

FIGURE 1

COMPRESSION PROFILE OF COATED PARTICLES

WITH VS. WITHOUT COMPRESSION COATING

HARDNESS (STRONG-COBB UNITS)

COMPRESSIONAL FORCE (kN)

□ DIVALPROEX SODIUM PARTICLES WITHOUT COATING

+ DIVALPROEX SODIUM PARTICLES WITH COATING (EXAMPLE 1)

11